# EUROPEAN PATENT APPLICATION

(11) **EP 4 497 823 A1**
(43) Date of publication of application: **29.01.2025**
(21) Application number: 23187384.5
(22) Date of filing: 24.07.2023
(51) Int. Cl.: C12N 9/00, C12M 1/40, B01L 3/00, C08J 11/10, G01N 33/50

(54) **A METHOD FOR SCREENING THE POLYMER-DEGRADING ENZYMES**

(71) Applicant: Vilnius University, 01513 Vilnius (LT)
(72) Inventor: Meskys, Rolandas, Vilnius (LT); Urbeliene, Nina, Vilnius (LT); Gasparaviciute, Renata, Vilnius (LT); Gudiukaite, Renata, Vilnius (LT)
(74) Representative: Petniunaite, Jurga

(57) **Abstract**

The present invention is provided methods of screening for polymer-degrading enzymes and microorganisms, enzymes or microorganisms variants which display increased activity towards native or/and synthetic polymers.

## Description

### FIELD OF THE INVENTION

This invention relates to the field of biochemistry, biotechnology and microbiology, more specifically to selection of polymer-degrading enzymes.

### BACKGROUND OF THE INVENTION

Many of the unnatural and natural polymers used in industry pose an environmentally important waste disposal problem. An important role in the biodegradation of polymers plays various enzymes. Biodegradation of polymeric materials are the process when polymers are broken down into fine fractions by microbial communities and abiotic factors present in the environment. The polymer backbones are broken down by a variety of hydrolytic enzymes produced by various biodegrading microorganisms. This leads to a gradual decrease in the molecular weight of the polymer. The polymers may be natural or synthetic.

Natural polymers are defined as materials that widely occur in nature or are extracted from plants or animals. Some of the examples of natural polymers are proteins (collagen, silk, and wool), polynucleic acids (DNA/RNA), cellulose, polysaccharide (cellulose, pectin, agar, starch, and etc.), lignins and etc. The most common synthetic polymers are polyester, PP, PET, PS, PUR, PVC, PE, PLA, acetyl-cellulose etc (Chen, CC., 2020).

Natural polymers may be degraded in contact with body fluids and tissues by several enzymes either by oxidation or hydrolysis. The hydrolytic enzymes (e.g. phosphatases, esterases, proteases/peptidases) are present in abundance and hydrolyze the substrates to help in the absorption of nutrients and solutes. These enzymes are also crucial in degrading polymers by hydrolysis resulting in the elevated degradation rate *in vivo.* Interestingly, microorganisms have evolved to use these natural polymers could degrade additionally synthetic plastic polymers. For example: 1) PET is converted by a PET-digesting enzyme (PETase) to mainly form mono(2-hydroxyethyl) terephthalic acid (MHET), while producing trace amounts of bis(2-hydroxyethyl)-terephthalic acid (BHET) and terephthalic acid (TPA) as byproducts. Subsequently, MHET is further digested by an MHET-digesting enzyme (MHETase) to generate ethylene glycol (EG) and TPA, which are the monomers of PET and can be used for PET recycling or upcycling; 2) Some dehydrogenases hydrolysed polyethylene glycol and polypropylene glycol Polytetramethylene glycol; 3) Oxidases, dehydrogenases or hydrolases degraded polyvinyl alcohol, 4) Lipases and cutinases - Polycaprolactones;5) Polylactic acid is degraded by proteases and lipases (Banerjee, A., 2014).

However, most of the knowledge on the microbial ability to degrade synthetic plastics is based on a few bacteria representing only <0.1% of the estimated bacteria inhabiting the biosphere. Moreover, Zrimec et al. (2021) revealed 30.000 nonredundant enzyme homologues in data bases with the potential to degrade 10 different plastic types. Thus, enormous number of different microorganisms and enzymes for different polymers degradation already exist in nature and are yet to be explored. The simple, rapid and user friendly screening system is urgent needed to test they ability toward different polymers. The plastic biodegradation process shows the changes of plastic surface structure: changes in color, creation of cracks and holes, roughening of the surface and formation of microbial colonies over the surface. The change in mechanical properties of plastic is usually determined by measurement of weight loss is a commonly used and standardized method for the assessment of plastic biodegradation. However, the correct cleaning of the samples is important, since the release of soluble and volatile impurities can also cause the weight loss rather than the microbial biodegradation itself. Gel permeation chromatography (GPC) is a general method to analyse the Mw and number average molecular weight (Mn) of the plastics after treatment (Zhang et al., 2021). Moreover, absorbance change in FTIR, X-ray powder diffraction (XRD), differential scanning calorimetry (DSC), transmission electron microscopy (TEM) and scanning force microscopy (SFM) are suggested to deepen the understanding of degradation mechanism.

The scientific literature describes a number of methods for screening or selection of polymer degrading enzymes or microorganisms. Functional screening or selection of new enzymes is usually based on the cultivation of micro-organisms in nutrient media, enriched with appropriated substrates. "Screening of enzymes" refers to evaluation of every protein for the desired property, while "selection of enzymes" automatically eliminates non-functional variants. Selection or screening of enzymes can be performed from enzyme libraries *in vivo* or *in vitro.* Screening or selection *in vivo* means that the substrates used must be soluble and non-toxic to the organism. Synthetic plastics, including bioplastics are generally not soluble in water, which makes the preparation of plastic-containing media a fastidious, time-consuming, and labor-intensive task. Clearance assays are often used to screen for plastic-degradable enzyme activity. If the plastic in the medium breaks down, a clear halo appears. Two methods are often used to incorporate plastics into the medium: emulsification and the incorporation of semi-water soluble plastics. In emulsification, the plastics are dissolved in an organic solvent such as dichloromethane and mixed with surfactants and growth medium. The solvent is subsequently evaporated, resulting in the formation of small plastic droplets in the medium. The emulsification is not an easy method, it is difficult to reproduce, and it is difficult to maintain the stability of the final dispersion. It is difficult to homogeneously distribute plastics while ensuring a large surface area among water, nutrients, and microorganisms on solid media to produce an effective screening medium. It is also difficult to exclude solvents or surfactants that may have potential cytotoxicity. The emulsion particles are mainly inside the solid medium rather than on the surface. The bulk film method is simple to produce in contrast to the emulsion method but offers a limited surface area between microorganisms and the plastic sample, which decreases the biodegradation efficiency and increases the experimental duration. Due to the lack of preparation methods for selective media, studies on the screening of degrading microbes have been limited. (Shin, 2021; Kim 2017)

The other methods for screening of PET enzymes published by Heyde, S.A.H, et.al. in 2021 year. To create a more efficient screening platform in *E. coli*, they display anchors for fast and easy assaying of PETase activity. This method allows only recombinant enzymes to be found. It is only suitable for screening mutant libraries. The effectiveness of this method is low.

The Chromogenic Assay method for Carbohydrates active enzymes (CAZymes) is one of the screening methods for enzymes *in vitro* (Schückel, 2016). Two distinct substrate types were used for detection: Chromogenic Polymer Hydrogel (CPH) substrates (made from purified polysaccharides and proteins) and Insoluble Chromogenic Biomass (ICB) substrates (made from complex biomass materials). Both CPH and ICB substrates are provided in a 96-well high-throughput assay system. This method provide screening exclusively Carbohydrates active enzymes.

Our proposed screening method is versatile: any polymer compound can be used. The method is not limited by substrate solubility, temperature and, in some cases, the addition of organic solvents. Suitable for screening both aerobic and anaerobic micro-organisms, for screening oxidation-sensitive enzymes, for thermophilic enzymes, and for the study of enzymes produced by *in vitro* transcription-translation.

The closest analogue to the invention is described by Shin, G. and et.al. (2021). The authors used polymer film or spray-based screening methods in agar-plate media. The hallo formation indicated the activity of the depolymerases. Such a method cannot be used for thermophilic enzyme screening due to the physical properties of agar. In addition, the agar plate method is not compatible with organic solvents, so enzymes or micro-organisms resistant to organic solvents cannot be selected. The method described in the present invention has a much broader range of applications: any polymer, a wide range of temperature modes, a wide range of media, buffer and its additives can be used. The method allows the analysis of enzymes present in living microorganisms as well as purified/extracted enzymes, or microorganism lysates and extracts. Also, screening of enzymes synthesised by *in vitro* transcription-translation can be tested.

### SUMMARY OF THE INVENTION

This invention provides a method of selection or screening by disposing on polymers film a plurality of uncharacterized polymer-degrading activity of secretory cells, secreted cell product, cells lysates or purified enzymes. The present invention relates to selection process of large libraries of polypeptides during which polypeptides degrade the substrate polymers to its corresponding product compounds.

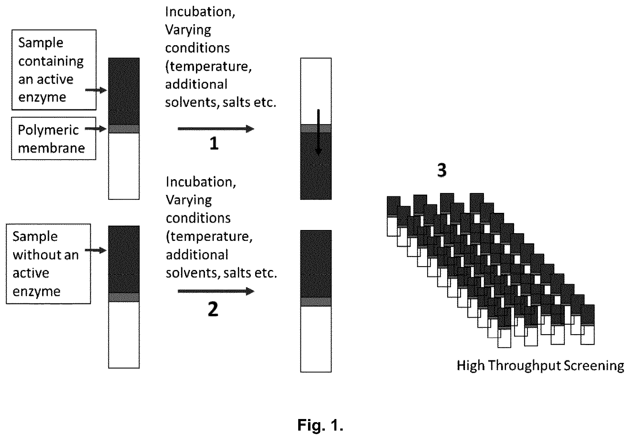

The invention provides a tubes or microplates with at least one polymer film partition for testing unknown cell-derived products or enzymes. The polymer film separates the upper and bottom tubes/wells cameras (Fig. 1). When the enzyme breaks the film the liquid passes from the upper reservoir to the bottom one.

### BRIEF DESCRIPTION OF FIGURES

The features of the invention, which is new and non-obvious, are given in the Claims. However, the invention may be best understood from the following detailed description of the invention, in which, without limiting the scope of the invention, exemplary embodiments of the invention are given in conjunction with the accompanying drawings, where:
**Fig. 1** depicts the principle schema of provided method: 1. the sample containing polymer degrading enzyme is added to upper reservoir of well and incubated at temperatures and conditions appropriate to the enzyme. If the enzyme hydrolyses the corresponding polymer, the sample liquid flows from the upper reservoir to the bottom. 2. The sample without polymer degrading enzyme is added to upper reservoir of well and incubated at temperatures and conditions appropriate to the enzyme. The sample liquid remains in the upper reservoir. 3. An illustration of the high throughput screening devices.

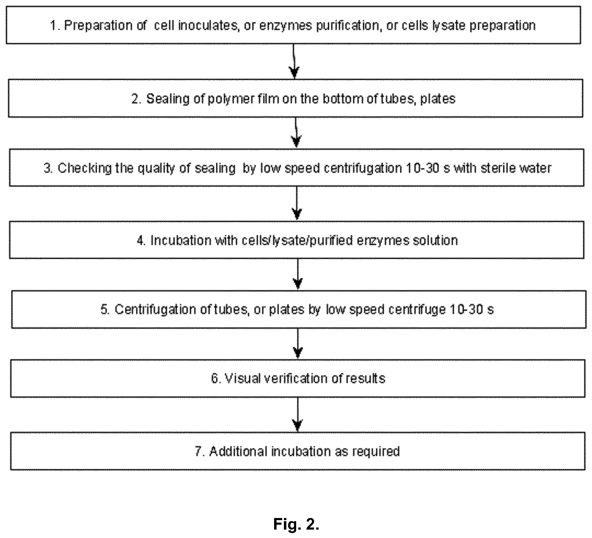

**Fig. 2** depicts flowchart illustrating a method for screening of polymer-degrading enzymes. The points 1-7 are described the procedure of screening.1. Preparation of tested samples: The testing of cells (inoculates) are cultivated in the nutrient media, or the tested enzymes are purified, or cells lysates are prepared according to the chosen protocol. 2. Sealing of polymer film: A device for detection of polymer degrading enzymes is manufactured via sealing of polymer film to the surface of tubes/microplates by using polymer sealers, or natural, or synthetic glues. 3. The checking of film sealing strength: sterile water is added into the tubes/microplates with sealed polymer film and after that tubes/microplates are centrifuged at low speed 10-30s. 4. Incubation with tested samples: the tested cells/enzymes/lysates solution are added into the checked tubes/microplates and samples are incubated with polymer films. The samples can be incubated indefinitely, preventing them from freeze-drying. 5. Polymer degrading activity detection: the tubes with samples are centrifuged at low speed 10-30s after appropriate incubation time. 6. Visual verification: The liquid entry to the bottom reservoir indicated polymer degrading activity. 7. Repeating of the incubation: if no activity is observed after the first check, the incubation can be extended and monitoring of activity can be performed an unlimited number of times.

### DETAILED DESCRIPTION OF THE INVENTION

The method for the detection of polymer-degrading enzymes shall consist of the following steps: a) preparation of cell inoculates, or purified enzymes, or cells lysate; b) incubation of sealed polymer with cells/lysate/purified enzymes solution and c) visual verification of results (Fig. 2).

A device for detection of polymer degrading enzymes may be manufactured via sealing of polymer film to the surface of tubes/plates by using polymer sealers, or natural, or synthetic glues. Additionally, the tubes or plates could be printed on 3D printer from the plastic of interest. An engraving plate includes a single or plurality of microwells.

The tested samples are incubated with polymer films at least 10 min, but can be incubated indefinitely, preventing them from freeze-drying. The function or secretory profile of the cell or cells is unknown.

The selected product is a secreted cell product and selected from the group consist hydrolases, depolymerases, esterases, lipases, cutinases, PETases, glycosidases, nucleases, peptidases, lyases, oxidoreductases.

This product is obtained from cell lysate or enzyme solution. It is obtained by *in vitro* transcription-translation reaction, varying the composition of reaction solution or by varying the temperature of sample incubation.

In some embodiments the wells of the plate contain at least one polymer.

The volume of reaction mixture may be at least 20 µl of analysed liquid with polymer film, being disposed in one of a plurality of microwells.

Polymer has contact with cells and cell-derived products.

In some embodiments the screening of the polymer-degrading enzymes in a cell culture may be cultivated in the nutrient media, for example LB, Bchl, mineral M9, Cosero, YPD and etc. Bacterial strains were loaded into microwells device with nutrient, sealed with a membrane and cultivated at appropriate temperature.

In another embodiments the screening of the polymer-degrading enzymes in a cell culture may be cultivated in the media under aerobic condition.

In another embodiments the screening of the polymer-degrading enzymes in a cell culture may be cultivated in the grow media under anaerobic condition.

In another embodiments the screening of the polymer-degrading enzymes may be performed directly in the *in vitro* transcription-translation reaction mixture.

In some embodiments the device may be used for optimisation of reaction mixture for degradation efficiently: buffer composition and optimal pH, detergents and concentration and of them, salts and concentration of them.

In another embodiments the device may be used for optimisation of reaction temperature or selected product is a product obtained by varying the temperature of sample incubation. The incubation temperature must not exceed the melting point of the polymer.

In some embodiments the device may be used for searching of enzymes degrading non-formatted film polymer. In this case, at least two polymers are used: one forming the film and the other added as a layer on top.

Alternatively, solution may to have stain or pH indicator, for example Phenol Red, Congo Red, Bromocresol Purple or Bromothymol Blue in all the cases mentioned above. The stain facilitates the monitoring of results.

The term polypeptide-degrading enzymes as used therein, unless otherwise specified, refers to a polypeptide that catalyses the irreversible hydrolytic degradation of polymer.

The term depolymerase as used therein, unless otherwise specified, refers to a polypeptide that catalyses the irreversible hydrolytic degradation of polymer.

The term "biological entity of interest" as used therein, unless otherwise specified, refers to enzymes catalyses irreversible hydrolytic degradation of polymers. Examples of enzymes are hydrolases, depolymerases, esterases, lipases, cutinases, PETases, glycosidases, nucleases, peptidases, lyases, oxidoreductases and etc. The source of enzymes can be cells, protein solution, tissues, blood, soil, water, sludge and etc.

As used herein, the term "polymer" means a natural or synthetic substance which has a molecular structure built up chiefly or completely from a large number of similar units bonded together.

"Screening of enzymes" refers to evaluation of every protein for the desired property, while "selection of enzymes" automatically eliminates non-functional variants. Selection or screening of enzymes can be performed from enzyme libraries *in vivo* or *in vitro.* The term "enzyme library" refers to a set of proteins or genes inserted into expression vectors or cells producing the enzymes. The enzyme library can be a metagenomic library or a mutant library. The metagenomic library can be constructed from various sources of environmental sample, such as soil, water, sludge, and etc. Specifically, the selected or screened enzyme may be of any enzyme group, for example, such as, hydrolases, depolymerases, esterases, lipases, cutinases, PETases, glycosidases, nucleases, peptidases, lyases, oxidoreductases and etc.

### EMBODIMENTS OF THE INVENTION

The following is information on specific examples describing implementation of the invention. Embodiments of the invention are provided for illustration, they do not limit the scope of the invention.

Examples of implementation of the invention:

### 1. Polycaprolactone (PCL) hydrolysis by cutinase.

Overexpression and purification of cutinase. The recombinant protein cutinase from *Streptomyces scabiei* 87.22 (Greičius, A, et.al. 2023) were overexpressed in E. *coli* strain BL21 (DE3). E. *coli* cells were grown in LB medium containing ampicillin (100 µg/mL) at 37°C with aeration. Protein expression was induced by adding IPTG to a final concentration of 0.5 mM at 0.6-1 OD₆₀₀, and cells were grown at 30 °C after induction for 4-18 h. Wet cell biomass from culture broth was suspended in the buffer A (50 mM potassium phosphate, pH 7.5), and disrupted by sonication for 2.5 min. A lysate was cleared by centrifugation at 15,000×*g* for 4 min. Cleared lysate was applied to Ni-NTA column (equilibrated with the buffer A). The column was washed with the buffer A, and the proteins were eluted with buffer A containing 500 mM imidazole. The active fractions were combined and dialyzed against the buffer B (50 mM potassium phosphate, pH 7.5), at 25 °C. All the purification procedures were performed at room temperature.

Film formation from PCL pellets. PCL Mw 45.000 and Mw 80.000 pellets were dissolved in chloroform to obtain 5 % w/v solution. The solution was mixed at room temperature until PCL pellets completely dissolved and a homogenous solution was obtained. Then, 4 ml of such a mixture was transferred to a glass Petri dish (5 cm diameter) and left to dry overnight (Khan et al., 2019). Intact films were used for further experiments.

Hydrolytic activity detection. PCL film was glued to the plate with wells and was staked on the standard PCR plate. So, the PCL film separates the top and bottom platewells. 200 µl of reaction mixture (50 mM potassium phosphate buffer, pH 7.5, 0.5 mM Phenol Red and cutinase 5 µg/ml) were added into top platewells. The top plate was sealed with adhesive aluminium seal to protect evaporation. The samples were incubated at RT (22-23 °C) up to 3 days. After incubation the plate with samples was centrifuged at low speed. The liquid entry from the top well to the bottom well indicates depolymerase activity in the sample. The hydrolytic activity of cutinase towards PCL Mₙ 80000 has been detected after 1 day incubation.

### 2. Polymer degradation rate dependence on enzyme concentration.

60 µl of reaction mixtures contains 50 mM potassium phosphate buffer, pH 7.5 and 0.9, 4.5, 9 or 45 µg of cutinase from *Streptomyces scabiei* were added into tubes. The tubes were sealed with polycaprolactone film from PCL Mw 80000. All reaction samples were tested in triplicate. The samples were incubated at RT (23 °C) up to 18h hours. The tubes with samples was centrifuged at low speed after 10, 15, 20, 25, 30, 40, 50 and 60 min. incubation time. The liquid entry to the bottom reservoir indicated depolymerase activity. The activity was detected after 15 min of incubation in the sample with 45 µg of cutinase, after 25 min in the sample with 9 µg of cutinase, after 50 min in the sample with 4.5 µg of cutinase and after 18 h in the sample with 0.9 µg of cutinase.

### 3. Screening of the organic solvent resistant polymer-degrading enzymes.

60 µl of reaction mixtures contains 50 mM potassium phosphate buffer, pH 7.5, 45 µg cutinase from *Streptomyces scabiei* and different quantity of ethanol (0%, 30% or 50% of ethanol) were added into tubes. The tubes were sealed with polycaprolactone film from PCL Mw 80000. All reaction samples were tested in triplicate. The samples were incubated at RT (23 °C) up to 2 hours. The tubes with samples was centrifuged at low speed after 10, 15, 20, 30, 60 and 120 min of incubation time. The liquid entry to the bottom reservoir indicated depolymerase activity. The activity was detected after 15 min of incubation in the sample without ethanol, after 20 min in the sample with 30% ethanol, and after 120 min in the sample with 50% of ethanol.

### 4. Screening of the polymer-degrading enzymes expressed in E. coli cells.

The recombinant proteins cutinase from *Streptomyces scabiei* 87.22 (Greičius, A, et.al. 2023) or TfCut2 cutinase (pEE075 Plasmid #176832, Addgene) were overexpressed in *E. coli* strain BL21 (DE3). E. *coli* cells were grown in LB medium containing ampicillin (100 µg/mL) at 37°C with aeration or anaerobically. Protein expression was induced by adding IPTG to a final concentration of 0.5 mM at 0.6-1 OD₆₀₀, and cells were grown at 20 °C after induction for 4-18 h. Than 200 µl of culture liquid was added to the well with sealed PCL (Mw 80.000) film bottom. The top plate was sealed with adhesive aluminium seal to protect evaporation. The samples were incubated at 30 °C up to 2 days. After incubation the plate with samples was centrifuged at low speed. The liquid entry from the top well to the bottom well indicates depolymerase activity in the sample. The activity of depolymerase was detected after 2 days of incubation at 23° (cutinase from *Streptomyces scabiei*) and at 55°C (TfCut2 cutinase). 5. Screening of the microorganisms which degrade polycaprolactone Mw 80000 film.

The soil microorganisms were cultivated into LB agar plate contained 1% polycaprolactone diol Mn 530. The microorganisms which showed hydrolysis zone onto PCL Mn530 agar were selected and cultivated in LB media at 30°C 20-24h. Than 500 µl of culture liquid was added to the well with sealed PCL film bottom. The top plate was sealed with adhesive aluminium seal to protect evaporation. The samples were incubated at 30 °C up to 3 days. After incubation the plate with samples was centrifuged at low speed. The liquid entry from the top well to the bottom well indicates depolymerase activity in the sample. The activity towards PCL 80000 film exhibited *Paenibacillus xylanexedens, Pseudomonas brassicacearum, Bacillus amyloliquefaciens* and *Moraxella osloensis* bacteria after 3-5 days incubation at 30°C temperature. 6. Detection of cellulose degradation microorganisms.

Cellulose film (MERCK-Millipore cellulose filter) was glued to the plate with wells and was staked on the standard PCR plate. So, the cellulose film separates the top and bottom plate wells. Micromycetes *Lomentospora prolificans* (Kal3); *Paraphaeosphaeria* (J61); *Trichoderma* sp. (GK71); *Galerina* sp. FN666098.1 (Kal2), *Fusarium oxysporum* (MOL2); *Alternaria alternate* (148); *Lithothelium* (95) were cultivated in nutrient media ( malt extract 3.5 g/l; yeast extract 2.5 g/l; KH₂PO₄ 2 g/l; MgSO₄·7H₂O 0.5 g/l; glucose 2 g/l, pH 5.5) at 25°C up to 4 days. Than 200 µl of extracellular liquid of micromycetes were added into top reservoir of plate wells. The top plate was sealed with adhesive aluminium seal to protect evaporation. The hydrolysis has been monitored up to 48 h incubation at RT (22-23 °C). After incubation the plate with samples was centrifuged at low speed. The liquid entry from the top well to the bottom well indicates cellulase activity in the sample.

The sample with *Trichoderma* sp. (GK71) extracellular liquid showed cellulose activity at RT after 1 day incubation.

### 7. Enzymatic degradation of polymer film at high temperature.

Overexpression and purification of cutinases from *Thermobifida fusca* KW3 (Genbank Acc. No. CBY05529.1 and CBY05530.1 ). The recombinant proteins were overexpressed in E. *coli* BL21(DE3) cells. E. *coli* biomass were grown in LB medium containing ampicillin (100 µg/mL) at 37°C with aeration. Protein expression was induced by adding IPTG to a final concentration of 0.5 mM at 0.6-1 OD₆₀₀, and cells were grown at 20 °C after induction for 18-20 h. Wet cell biomass from culture broth was suspended in the buffer A (50 mM potassium phosphate, pH 7.5), and disrupted by sonication for 2.5 min. A lysate was cleared by centrifugation at 15,000×*g* for 4 min. Cleared lysate was applied to Ni-NTA column (equilibrated with the buffer A). The column was washed with the buffer A, and the proteins were eluted with buffer A containing 500 mM imidazole. The active fractions were combined and dialyzed against the buffer B (50 mM potassium phosphate, pH 7.5), at 25 °C. All the purification procedures were performed at room temperature.

Degradation of PET or PLC film. The purified enzymes solutions were added into 2 ml glass vials. PCL (Mr80000) or PET film 0.013 mm (Goodfellow) was fixed on the vial's top by screws with center hole. The vials were placed (with top down) in other larger tubes. The samples were incubated 2-30 days at 50°C and 55°C. After incubation vials with samples was centrifuged at low speed. The liquid entry from the vials to the tubes indicates hydrolytic activity of the enzymes. Cutinases from *Thermobifida fusca* KW3 (Genbank Acc. No. CBY05529.1 and CBY05530.1) exhibited activity towards PCL (Mr80000) film after 2 days. Cutinase (CBY05530.1) showed activity towards PET film after 30 days of incubation at 55 °C temperature. 8. Degradation of PCL film, when enzyme is obtained by *in vitro* transcription-translation reaction.

The transcription-translation reaction was performed by using PURExpress^{®} In Vitro Protein Synthesis Kit (NEB). The reaction mix contained solution A, 10 µl, Solution B, 7.5 µl, nuclease-free H₂O, 3.5 µl and Template DNA (plasmid with *Thermobifida fusca* KW3 gene) 4 µl (50 ng/µl). The reaction mixtures were incubated 2 h at 37°C. 25 µl of transcription-translation reaction mixtures were added into tubes. The tubes were sealed with polycaprolactone film from PCL Mw 80000. All reaction samples were tested in triplicate. The samples were incubated at RT (23 °C) up to 48h hours. The tubes with samples was centrifuged at low speed after incubation time. The liquid entry to the bottom reservoir indicated depolymerase activity. The activity was detected after 48 h of incubation.

### Literature

1. Chen, CC., Dai, L., Ma, L. et al. Enzymatic degradation of plant biomass and synthetic polymers. Nat Rev Chem 4, 114-126 (2020). https://doi.org/10.1038/s41570-020-0163-6
2. Greicius, A.; Baliutavicius, T.; Lastauskiene, E.; Gudiukaite, R. Application of Milk Permeate as an Inducer for the Production of Microbial Recombinant Lipolytic Enzymes. Fermentation 2023, 9, 27. https://doi.org/10.3390/fermentation9010027
3. Heyde, S.A.H., Arnling Bååth, J., Westh, P. et al. Surface display as a functional screening platform for detecting enzymes active on PET. Microb Cell Fact 20, 93 (2021). https://doi.org/10.1186/s12934-021-01582-7
4. Khan, I., Nagarjuna, R.J., Dutta, R., Ganesan R. Enzyme-embedded degradation of poly(ε-caprolactone) using lipase-derived from probiotic Lactobacillus plantarum, ACS Omega 4 (2019) 2844-2852, https://doi.org/10.1021/acsomega.8b02642.
5. Kim, M. Y., Kim, C., Moon, J., Heo, J., Jung, S. P. and Kim, J. R. J. Microbiol. Biotechnol., 2017, 27, 342-349, doi.org/10.4014/jmb.1610.10015.
6. Pathak, V.M., Navneet Review on the current status of polymer degradation: a microbial approach. Bioresour. Bioprocess. 4, 15 (2017). https://doi.org/10.1186/s40643-017-0145-9
7. Shin, G., Park, S. A., Koo, J. M., Kim, M., Lee, M., Jegal, J., et al. (2021). A microspray-based high-throughput screening system for bioplastic-degrading microorganisms. Green Chem. 23, 5429-5436. doi: 10.1039/d1gc01916c
8. US 10,865,434 B2
9. US 8,865,479 B2

## Claims

1. A method for screening at least one biological entity, such as hydrolases, depolymerases, esterases, lipases, cutinases, PETases, glycosidases, nucleases, peptidases, lyases, oxidoreductases, in a sample using a device comprising a bottom polymer film defining a surface of plurality of microwells, **characterised in that** the method comprises: i) contacting of at less 20 µl volume of analysed liquid with polymer film, being disposed in one of a plurality of microwells, ii) contacting of polymers with cells and cell-derived products.

2. The method of claim 1, wherein the at least one selected product is a secreted cell product.

3. The method of claim 1, wherein the at least one selected product is obtained from cell lysate.

4. The method of claim 1, wherein the at least one selected product is obtained by *in vitro* transcription-translation reaction.

5. The method of claim 1, wherein the at least one selected product is obtained from enzyme solution.

6. The method of claim 2-5, wherein the at least one product is selected from the group consist hydrolases, depolymerases, esterases, lipases, cutinases, PETases, glycosidases, nucleases, peptidases, lyases, oxidoreductases.

7. The method of claim 2-5, wherein the at least one product is selected under aerobic condition.

8. The method of claim 2-5, wherein the at least one product is selected under anaerobic condition.

9. The method of claim 1, wherein the at least one selected product is a product obtained by varying the composition of reaction solution.

10. The method of claim 1, wherein the at least one selected product is a product obtained by varying the temperature of sample incubation.
